# EUROPEAN PATENT APPLICATION

(11) **EP 4 250 306 A1**
(43) Date of publication of application: **27.09.2023**
(21) Application number: 21894718.2
(22) Date of filing: 18.11.2021
(51) Int. Cl.: G16H 10/00, A61B 5/00, G06Q 50/10

(54) **BIOLOGICAL INFORMATION INTERPRETATION SUPPORT DEVICE, BIOLOGICAL INFORMATION INTERPRETATION SUPPORT METHOD, AND BIOLOGICAL INFORMATION INTERPRETATION SUPPORT PROGRAM**

(30) Priority: 18.11.2020 JP 2020191771
(71) Applicant: Fukuda Denshi Co., Ltd., Tokyo 113-8483 (JP)
(72) Inventor: FUKATANI, Kyohei, Tokyo 113-8483 (JP); KUWAYAMA, Takashi, Tokyo 113-8483 (JP); YASUNAGA, Fumiya, Tokyo 113-8483 (JP); ISODA, Yusuke, Tokyo 113-8483 (JP); ISHII, Yoshihiro, Tokyo 113-8483 (JP)
(74) Representative: Eisenführ Speiser
(86) International application number: PCT/JP2021/042421
(87) International publication number: WO 2022/107846

(57) **Abstract**

A biological information interpretation support device which includes an information output unit which displays, on a display screen, biological information and/or information pertaining to the result of analysis thereof; a request reception output unit which displays, on the display screen, an interpretation request reception part for receiving an interpretation request operation for the biological information; a designation reception unit which receives a designation of a feedback item that is requested as an interpretation result of the biological information when the interpretation request operation is made; and a transmission unit which transmits an interpretation request in which the designation of the feedback item is added to the biological information and/or the information pertaining to the result of analysis.

## Description

### Technical Field

The present invention relates to a biological information interpretation support apparatus, a biological information interpretation support method, and a biological information interpretation support program each supporting an interpretation of biological information.

### Background Art

To diagnose the health conditions of a living body, for example, a human patient, biological information in the living body, such as, for example, an electrocardiographic waveform and a blood pressure and pulse waveform, is measured by examination apparatuses such as an electrocardiograph and a blood pressure and pulse wave meter. The biological information measured in the above-described manner is used as valuable examination information at a clinic or the like (Patent Literature (hereinafter referred to as "PTL") 1).

### Citation List

### Patent Literature

PTL 1
Japanese Patent Application Laid-Open No. 2014-061180

### Summary of Invention

### Technical Problem

The biological information measured by the examination apparatuses as described above includes individual differences and may not necessarily be clear in characteristics. For this reason, it may be difficult for a medical worker at a clinic, such as a nurse, a medical laboratory scientist, and a doctor, to interpret biological information. Further, even when biological information is interpreted, it may be difficult to derive an accurate examination result. For example, electrocardiographic waveforms include individual differences, some of the waveforms are not necessarily clear in characteristics, and it may be difficult to interpret the waveforms and to derive an examination result. The same applies to a medical worker at a medical checkup office (hereafter referred to as "MC office") in which a medical checkup is conducted.

In such a case, a medical worker at a clinic or an MC office requests an expert on the examination of biological information to interpret biological information. For example, in the case of an electrocardiographic waveform, a medical worker requests a doctor specialized in the circulatory system (a cardiovascular specialist) or the like to interpret the electrocardiographic waveform. However, it is difficult for a medical worker at a clinic or an MC office to feel free to request an interpretation unless the medical worker has an acquaintance who is an expert on the examination of biological information. This may lead to hesitation in requesting an interpretation and further to hesitation in performing measurement with an examination apparatus such as an electrocardiograph. For this reason, a system capable of supporting a medical worker at a clinic or an MC office in requesting an expert on the examination of biological information to interpret biological information is desired.

An object of the present invention is to provide a biological information interpretation assistance apparatus, a biological information interpretation support method, and a biological information interpretation support program each capable of supporting a request for an interpretation of biological information.

### Solution to Problem

A biological information interpretation support apparatus according to the present invention includes:
an information output section that causes a display screen to display biological information and/or information on an analysis result of the biological information;
a request reception output section that causes the display screen to display an interpretation request reception section that receives an interpretation request operation for the biological information;
a designation reception section that receives designation of a feedback item required as an interpretation result of the biological information during the interpretation request operation; and
a transmission section that transmits an interpretation request in which the designation of the feedback item is added to the biological information and/or the information on the analysis result.

Further, a biological information interpretation support method according to the present invention includes:
causing a display screen to display biological information and/or information on an analysis result of the biological information;
causing the display screen to display an interpretation request reception section that receives an interpretation request operation for the biological information;
receiving designation of a feedback item required as an interpretation result of the biological information during the interpretation request operation; and
transmitting an interpretation request in which the designation of the feedback item is added to the biological information and/or the information on the analysis result.

Further, a biological information interpretation support program according to the present invention causes a computer to execute the biological information interpretation support method described above.

### Advantageous Effects of Invention

According to the present invention, it is possible to support a request for an interpretation of biological information.

### Brief Description of Drawings

FIG. 1 is a block diagram provided for describing a biological information interpretation support system including a biological information interpretation support apparatus according to an embodiment of the present invention;
FIG. 2 is a sequence diagram provided for describing a biological information interpretation support method that is performed in the biological information interpretation support system illustrated in FIG. 1;
FIG. 3 illustrates a display screen of a request terminal in the biological information interpretation support system illustrated in FIG. 1, indicating an exemplary electrocardiogram and an exemplary analysis result;
FIG. 4 illustrates the display screen of the request terminal in the biological information interpretation support system illustrated in FIG. 1, indicating an example in which a heart sound waveform and a blood pressure and pulse waveform are described together with an electrocardiogram and an analysis result.
FIG. 5 illustrates the display screen of the request terminal in the biological information interpretation support system illustrated in FIG. 1, indicating an example of a feedback item designation section;
FIG. 6 illustrates the display screen of the request terminal in the biological information interpretation support system illustrated in FIG. 1, indicating an example of an interpretation request execution section;
FIG. 7 illustrates the display screen of the request terminal in the biological information interpretation support system illustrated in FIG. 1, indicating an example of a request destination reception section;
FIG. 8 illustrates a display screen of a reference terminal in the biological information interpretation support system illustrated in FIG. 1, indicating an example of an interpretation result input section;
FIG. 9 illustrates the display screen of the reference terminal in the biological information interpretation support system illustrated in FIG. 1, indicating an example in which parts of an electrocardiogram are enlarged; and
FIG. 10 illustrates the display screen of the reference terminal in the biological information interpretation support system illustrated in FIG. 1, exemplifying a blood pressure and pulse waveform for an interpretation together with an electrocardiogram, an analysis result, a heart sound waveform, and a blood pressure and pulse waveform.

### Description of Embodiment

Hereinafter, an embodiment of the present invention will be described with reference to the accompanying drawings.

### [Biological Information Interpretation Support System]

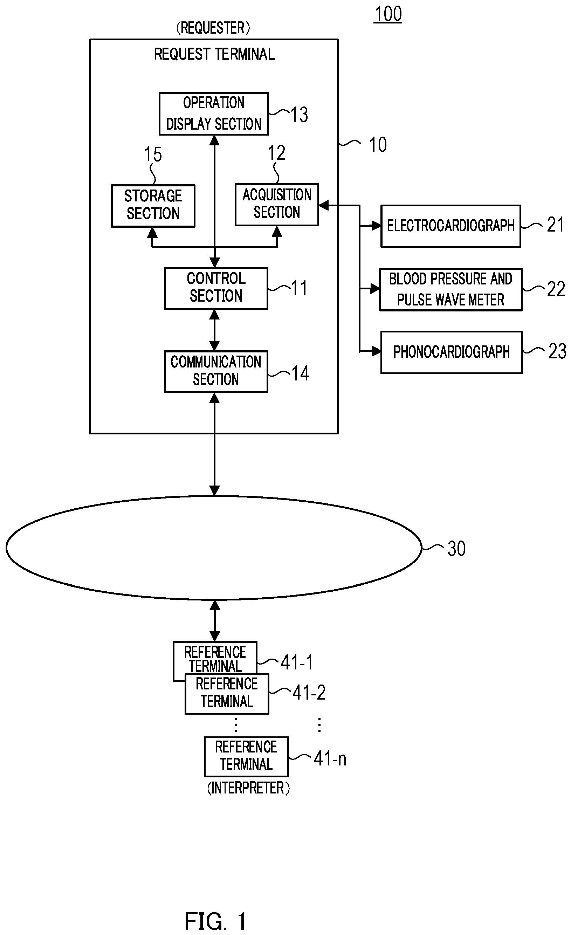

FIG. 1 is a block diagram provided for describing biological information interpretation support system 100. Biological information interpretation support system 100 includes request terminal 10, network 30, and reference terminals 41-1 to 41-n, where n is a natural number.

Request terminal 10 and reference terminals 41-1 to 41-n are connected to each other via network 30. Further, electrocardiograph 21, blood pressure and pulse wave meter 22, and phonocardiograph 23, which are exemplary measurement apparatuses that measure a biological waveform that is biological information of a patient (living body), are connected to request terminal 10. Note that, although biological waveforms such as an electrocardiographic waveform, a blood pressure and pulse waveform, and a heart sound waveform are exemplified as the biological information, the biological waveform is not limited thereto and may be, for example, an electroencephalogram or the like. Further, the biological information may be information indicating a dynamic state of a living body, such as, for example, oxygen saturation (SpO₂), information indicating a time-series change in expiratory flow, or the like.

When a biological waveform of a patient is measured by using electrocardiograph 21, blood pressure and pulse wave meter 22, and phonocardiograph 23, the measured biological waveform is inputted into request terminal 10. Then, as described later, a medical worker (requester) at a clinic or an MC office request an expert (interpreter) on examination of the measured biological waveform to interpret the measured biological waveform by using request terminal 10.

Note that, the requester is not limited to a medical worker, but may be, for example, a family member (relative) of a patient, or the like. Further, although the interpreter is desirably a doctor familiar with an interpretation of biological information, the interpreter may also be, for example, an engineer of a company that produces a measurement apparatus for measuring biological information, a medical laboratory scientist who uses the measurement apparatus, or the like as long as the engineer, the medical laboratory scientist, or the like is familiar with an interpretation of biological information. For example, in a case where the biological information is an electrocardiographic waveform, the doctor familiar with an interpretation of biological information falls under a cardiovascular specialist or the like.

Here, electrocardiograph 21 is an apparatus that determines an electrocardiographic waveform by measuring a minute electromotive force generated along with the activity of the heart with an electrode. Among electrocardiographs 21, a Holter electrocardiograph (including a measurement terminal and an analysis apparatus) is an apparatus capable of measuring an electrocardiographic waveform for a long time (for example, 24 hours or the like).

Further, blood pressure and pulse wave meter 22 is an apparatus that measures a blood pressure and pulse wave form by measuring the speed of the pulsation of blood flowing in the blood vessel and blood pressure.

Further, phonocardiograph 23 is an apparatus that measures a sound (heart sound), generated by the heart that is beating, for example, by using a heart sound microphone.

Note that, since a polysomnography apparatus that examines a sleep apnea syndrome (SAS) measures an electrocardiographic waveform, a blood pressure and pulse waveform, oxygen saturation, an expiratory flow, or the like, the measurement apparatus used in the present embodiment may be an apparatus capable of measuring such a plurality of items.

Electrocardiograph 21, blood pressure and pulse wave meter 22, and phonocardiograph 23 may have an automatic analysis function whereby electrocardiograph 21, blood pressure and pulse wave meter 22, and phonocardiograph 23 analyze biological waveforms of a measured electrocardiographic waveform, a measured blood pressure and pulse waveform, and a measured heart sound waveform, and output an analysis result of the biological waveforms. For example, electrocardiograph 21 detects division points (such as P, QRS, and T) in an electrocardiographic waveform, determines measurement values (such as R-R interval, ST level, PQ, QRS width, QT, or the like), decides an analysis result based on the determined measurement values and a determination condition set in advance, and outputs the decided analysis result. Note that, such an automatic analysis function may not be provided on a side of electrocardiograph 21, blood pressure and pulse wave meter 22, and phonocardiograph 23, but may be provided on a side of request terminal 10 and, in Variation 1 to be described later, may be provided on a side of a cloud server.

### [Request Terminal]

Request terminal 10 is an example of the biological information interpretation support apparatus in the present invention. Request terminal 10 is a computer and is disposed at, for example, a clinic or an MC office together with electrocardiograph 21, blood pressure and pulse wave meter 22, or the like described above. Note that, request terminal 10 is not limited to a computer, but may be a portable terminal, for example, a smartphone or a tablet terminal, that has a wireless communication function and is portable.

Specifically, request terminal 10 includes control section 11, acquisition section 12, operation display section 13, communication section 14, and storage section 15.

Control section 11 includes, although detailed illustration is omitted, a processor such as a central processing unit (CPU), and memories such as a random access memory (RAM) and a read only memory (ROM), or the like.

Control section 11 controls the operations of acquisition section 12, operation display section 13, communication section 14, and storage section 15 within request terminal 10.

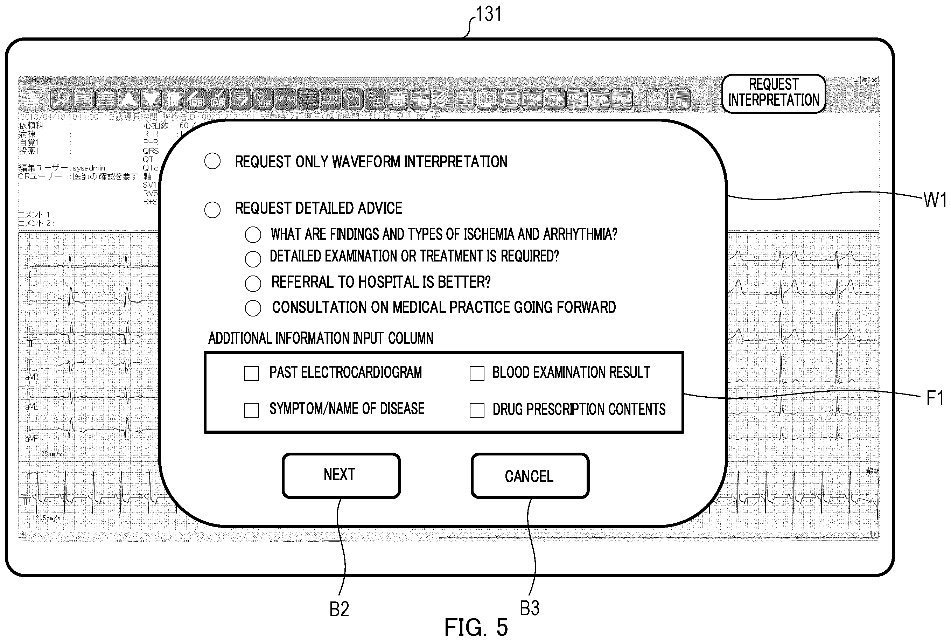

Further, control section 11 may have an additional information generation function of generating additional information (see FIG. 5) for supporting an interpretation request of a requester. For example, as described later, storage section 15 includes information of a patient (age, sex, physical information such as weight and height, past surgical history, medication information, situation of present disease, morbidity situation of other diseases, or the like), past biological waveform data of a patient, and biological waveform data of a family member(s) and/or a close relative(s) (relative(s)) of a patient. Control section 11 may be configured to be capable of generating additional information based on the information described above when an interpretation request is made, and transmitting the generated additional information to a request destination.

Acquisition section 12 acquires a biological waveform or the analysis result described above from electrocardiograph 21, blood pressure and pulse wave meter 22, and phonocardiograph 23. A requester performs a first diagnosis of the biological waveform based on the acquired biological waveform and analysis result, and further requests an interpreter to interpret the acquired biological waveform and analysis result.

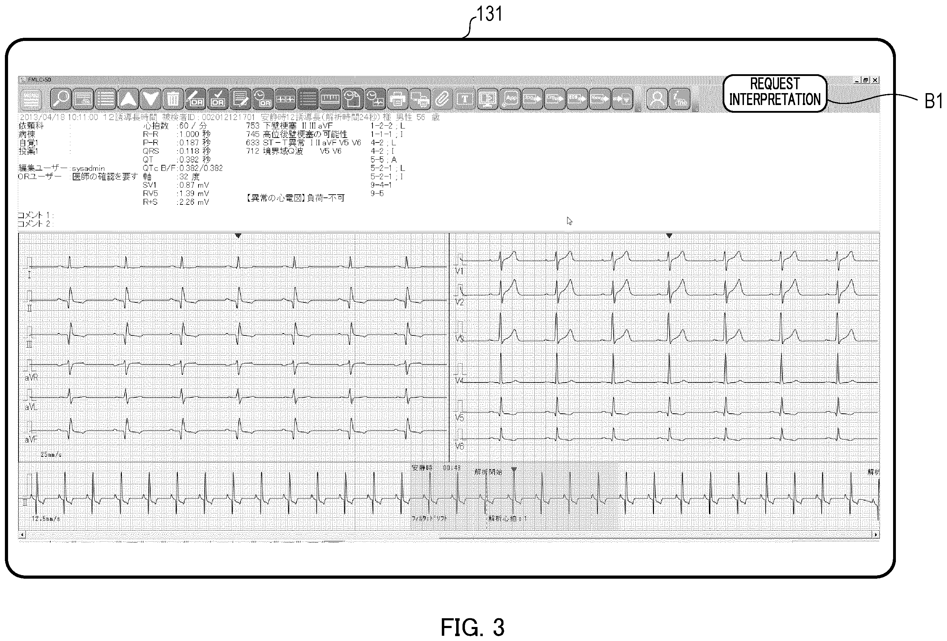

Operation display section 13 includes display screen 131 (see FIG. 3 or the like), causes display screen 131 to display an operation screen, and receives various input operations by a requester. Operation display section 13 may be formed of, for example, an operation section, such as a keyboard and a mouse, and a display section, such as a liquid crystal display, or may be formed of an operation section and a display section, such as a liquid crystal display with a touch screen.

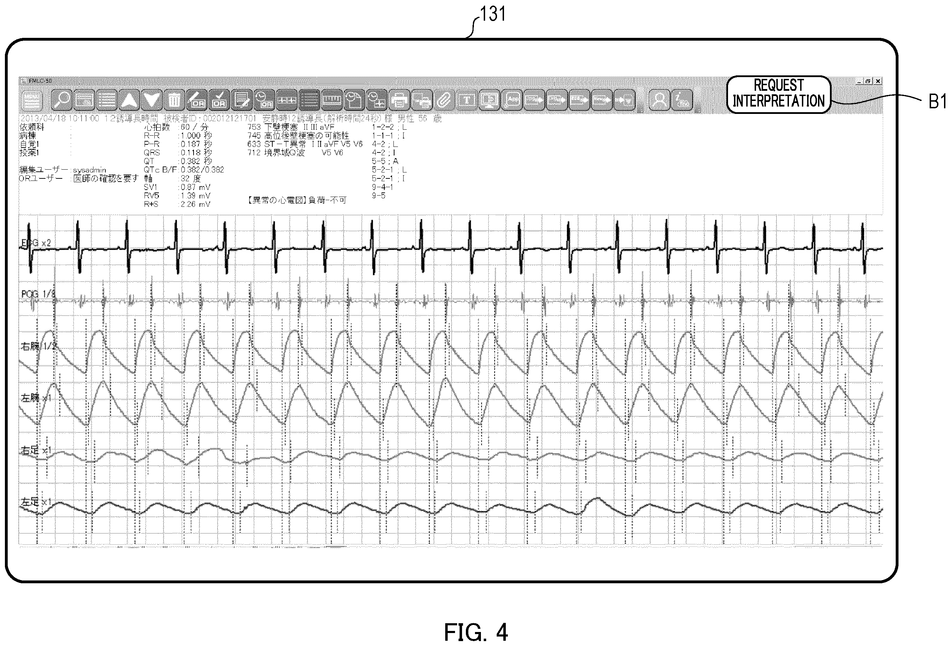

Operation display section 13 described above functions as an information output section that causes display screen 131 to display information on a biological waveform and/or an analysis result thereof (see FIGS. 3 and 4 to be described later). Further, operation display section 13 also functions as a request reception output section that causes display screen 131 to display an interpretation request reception section (operation button B1 of "REQUEST INTERPRETATION" illustrated in FIGS. 3 and 4) for receiving an interpretation request operation for a biological waveform. Further, operation display section 13 functions as a designation reception section that receives designation of a feedback item required as an interpretation result of a biological waveform during an interpretation request operation (see FIG. 5 to be described later).

Here, the designation reception section causes display screen 131 to display a feedback item designation section for receiving designation of a feedback item (see FIG. 5 to be described later).

Specifically, the designation reception section causes the feedback item designation section to display a feedback item that is designatable (see FIG. 5 to be described later).

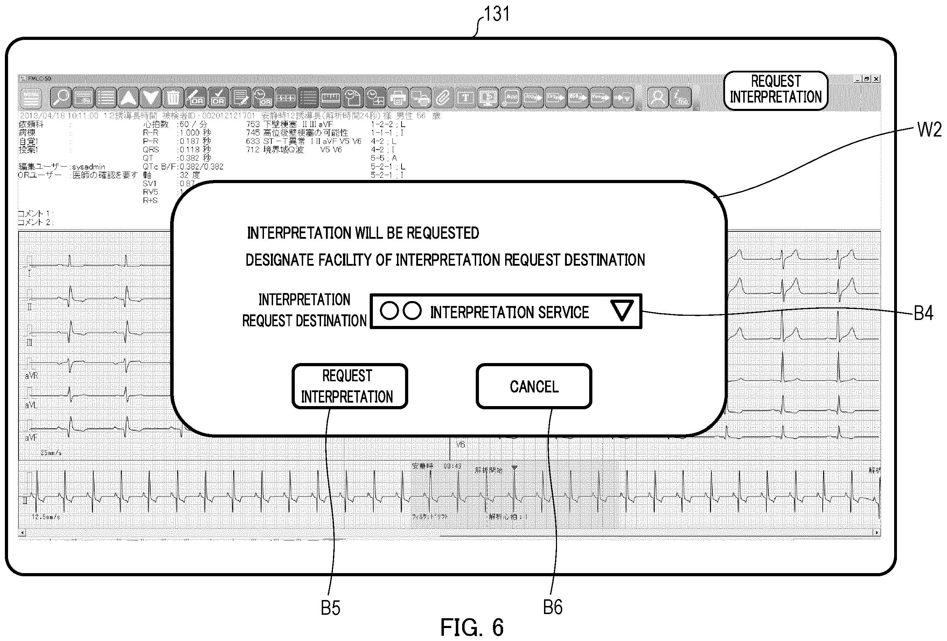

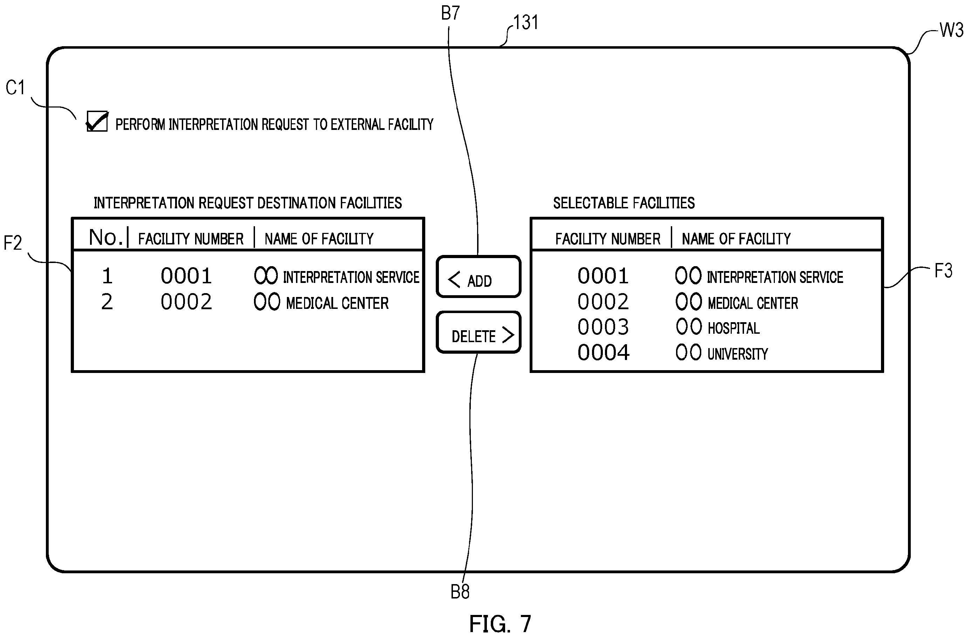

Further, the designation reception section may cause the feedback item designation section to be displayed after reception of an interpretation request operation. At this time, the designation reception section may cause the feedback item designation section and a request destination reception section (see FIGS. 6 and 7 to be described later), which is for receiving designation of a request destination of the interpretation request, to be sequentially displayed in accordance with the reception of the interpretation request operation. In this case, the designation reception section may cause the request destination reception section to display a plurality of request destinations, which is in a state in which the plurality of request destinations is allowed to receive the interpretation request, and may receive the designation of the request destination among the plurality of request destinations having been displayed.

Further, the designation reception section may cause the feedback item designation section to display an input column that receives an input of additional information including at least one of a history, a symptom, a prescription, and/or a chief complaint which are related to a living body for which a biological waveform is acquired. Further, the designation reception section may also cause the feedback item designation section to display an input column that receives an input of the additional information of a group (for example, family, relatives, region, or the like) to which a living body for which a biological waveform is acquired belongs. Examples of the history include a medical history (chief complaint, present medical history, past medical history, family history, social history, or the like), an examination history, or the like.

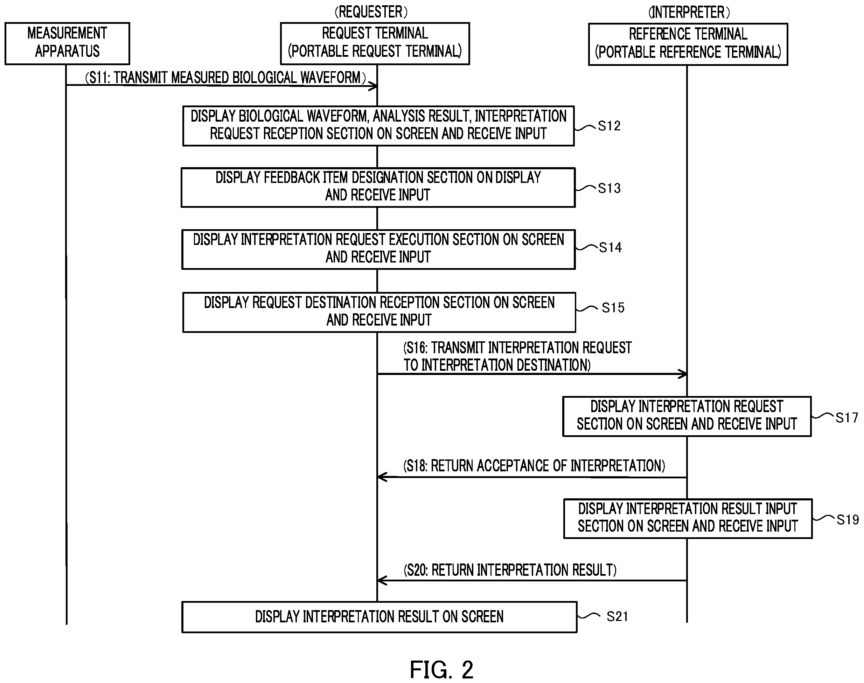

Note that, the information output section, interpretation request reception section, and request reception output section described above will be described together with the designation reception section later with reference to FIGS. 2 to 7.

Communication section 14 communicates with reference terminals 41-1 to 41-n via network 30. Communication section 14 functions as a transmission section that transmits an interpretation request, in which designation of a feedback item, additional information, and the like are added to the information on a biological waveform and an analysis result (information on a biological waveform or an analysis result) described above, to reference terminals 41-1 to 41-n. Further, communication section 14 also functions as a reception section that receives an interpretation result of a biological waveform from reference terminals 41-1 to 41-n.

Storage section 15 stores a biological waveform, an analysis result, an interpretation request, an interpretation result, and the like that are described above. Storage section 15 is formed of, for example, a non-volatile semiconductor memory (so-called flash memory), a hard disk drive (HDD), and the like. Further, storage section 15 may also accumulate information of a patient (for example, age, sex, physical information such as weight and height, past surgical history, medication information, situation of present disease, morbidity situation of other diseases, or the like), past biological waveform data of a patient, and biological waveform data of a family member(s) and/or a close relative(s) of a patient.

### [Network]

Network 30 is a communication network such as a local area network (LAN) and a telephone line. Note that, network 30 is not limited to a LAN and a telephone line, but may be a wide area network (WAN) or the like, and further may be wireless or wired.

### [Reference Terminal]

Reference terminal s 41-1 to 41-n are computers and are di sposed at a facility to which an interpreter who performs an interpretation of a biological waveform belongs (for example, a hospital specializing in the circulatory system), or the like. Note that, reference terminals 41-1 to 41-n are not limited to computers, but may be portable terminals, for example, smartphones or tablet terminals, that have a wireless communication function and are portable.

An interpretation request for a biological waveform from a requester arrives at one or a plurality of reference terminals among reference terminals 41-1 to 41-n. Then, the interpreter(s) who own(s) the reference terminal(s) at which the request has arrived return(s) acceptance/non-acceptance of the interpretation request and then, in the case of the acceptance of the interpretation request, performs the interpretation of the biological waveform for which the interpretation request has been made by request terminal 10 of the requester.

### [Biological Information Interpretation Support Method]

As described above, biological information interpretation support system 100 includes request terminal 10, network 30, and reference terminals 41-1 to 41-n. A biological information interpretation support method (a biological information interpretation support program) that is performed in biological information interpretation support system 100 having such a configuration will be described with reference to FIGS. 3 to 10 together with FIG. 2.

Note that, since the procedure is substantially the same between in a case where a requester at a clinic requests an interpretation and in a case where a requester at an MC office requests an interpretation, a description will be given, unless otherwise specified, without distinguishing between these requesters.

FIG. 2 is a sequence diagram provided for describing a biological information interpretation support method that is performed in biological information interpretation support system 100 illustrated in FIG. 1.

### (Step S11)

When a requester measures a biological waveform of a patient by using a measurement apparatus (such as electrocardiograph 21, blood pressure and pulse wave meter 22, and phonocardiograph 23), the measured biological waveform is transmitted to request terminal 10. At this time, the biological waveform and an analysis result thereof are transmitted to request terminal 10 in a case where the measurement apparatus has the automatic analysis function.

### (Step S12)

Request terminal 10 (information output section) causes display screen 131 to display the biological waveform. In a case where the measurement apparatus has the automatic analysis function, request terminal 10 may cause display screen 131 to display the biological waveform and information on the analysis result of the biological waveform or may cause display screen 131 to display the information on the analysis result of the biological waveform without causing the biological waveform to be displayed. In a case where the measurement apparatus does not have the automatic analysis function, for example, request terminal 10 may perform an automatic analysis of the biological waveform and may cause display screen 131 to display the biological waveform and the information on the analysis result of the biological waveform. Further, it may also be configured such that request terminal 10 transmits a biological waveform to an analysis-dedicated apparatus, the analysis-dedicated apparatus analyzes the biological waveform, and request terminal 10 receives an analysis result from the analysis-dedicated apparatus and then causes display screen 131 to display the biological waveform and the information on the analysis result of the biological waveform.

Further, request terminal 10 (request reception output section) causes display screen 131 to display an interpretation request reception section (operation button B1 of "REQUEST INTERPRETATION" illustrated in FIGS. 3 and 4) for receiving an interpretation request operation for a biological waveform.

FIG. 3 illustrates display screen 131 of request terminal 10 in biological information interpretation support system 100, indicating an exemplary electrocardiogram and an exemplary analysis result. In FIG. 3, an analysis result is displayed on the upper side, and an electrocardiogram (electrocardiographic waveform) that is a biological waveform is displayed on the lower side. Further, in FIG. 3, operation button B1 of "REQUEST INTERPRETATION" is disposed on the upper right side. When operation button B1 is clicked, the processing proceeds to next step S13.

Further, FIG. 4 illustrates display screen 131 of request terminal 10 in biological information interpretation support system 100, indicating an example in which a heart sound waveform and a blood pressure and pulse waveform are described together with an electrocardiogram and an analysis result. In FIG. 4, an analysis result is displayed on the upper side, and an electrocardiogram (electrocardiographic waveform), a heart sound waveform, and a blood pressure and pulse waveform, which are biological waveforms, are displayed on the lower side. In FIG. 4, the electrocardiographic waveform is marked as "ECG", the heart sound waveform is marked as "PCG", and the blood pressure and pulse waveform is marked as "right arm", "left arm", "right foot" and "left foot" that indicate the measured portions. Further, operation button B1 of "REQUEST INTERPRETATION" is also disposed in the upper right side in FIG. 4. When operation button B1 is clicked, the processing proceeds to next step S13.

### (Step S13)

When operation button B1 of "REQUEST INTERPRETATION" illustrated in FIGS. 3 and 4 is clicked, request terminal 10 (designation reception section) causes display screen 131 to display feedback item designation window (feedback item designation section) W1 for receiving designation of a feedback item (see FIG. 5). Then, request terminal 10 (designation reception section) receives designation of a feedback item that is requested as an interpretation result of a biological waveform during the interpretation request operation.

FIG. 5 illustrates display screen 131 of request terminal 10 in biological information interpretation support system 100, indicating an example of feedback item designation window W1. As illustrated in FIG. 5, feedback item designation window W1 displays, for receiving designation of a feedback item, radio buttons "o" and displays feedback items next to the radio buttons "∘". A requester can easily select a feedback item by simply clicking a radio button "∘".

FIG. 5 exemplifies "REQUEST ONLY WAVEFORM INTERPRETATION RESULT" and "REQUEST DETAILED ADVICE" as large items for feedback items. Further, as small items for "REQUEST DETAILED ADVICE", FIG. 5 exemplifies "WHAT ARE FINDINGS AND TYPES OF ISCHEMIA AND ARRHYTHMIA?", "DETAILED EXAMINATION OR TREATMENT IS REQUIRED?", "REFERRAL TO HOSPITAL IS BETTER?", "CONSULTATION ON MEDICAL PRACTICE GOING FORWARD", or the like. These large and small items are exemplary, and the feedback items are not limited thereto. For example, "WHETHER TRANSPORTATION TO SPECIALIZED MEDICAL INSTITUTION IS REQUIRED OR NOT" or the like may be a feedback item.

Further, for example, in a case where request terminal 10 is disposed at an MC office, a feedback item(s) different from that/those in a case where request terminal 10 is disposed at a clinic may be required. Specifically, in a case where first screening of an electrocardiographic waveform is performed at an MC office, "WHETHER FIRST SCREENING IS APPROPRIATE OR NOT", "WHETHER RESULT OF FIRST SCREENING IS APPROPRIATE OR NOT", or the like may be a feedback item.

Further, request terminal 10 (designation reception section) may cause feedback item designation window W1 to display additional information input column F1 that receives an input of additional information including at least one of a history, a symptom, a prescription, and/or a chief complaint which are related to a living body for which a biological waveform is acquired.

For example, as illustrated in FIG. 5, additional information input column F1 displays checkboxes "□" and input items for additional information. Here, although "PAST ELECTROCARDIOGRAM", "BLOOD EXAMINATION RESULT", "SYMPTOM/NAME OF DISEASE" and "DRUG PRESCRIPTION CONTENTS" are displayed as the input items for the additional information, these are exemplifications of the history, symptom, prescription, and chief complaint, and the input items are not limited thereto. Further, the history may include, for example, a medical history (chief complaint, present medical history, past medical history, family history, social history, or the like), examination history, or the like.

Then, when these checkboxes are checked, relevant additional information can be inputted or data of additional information can be attached. For example, in a case where the checkbox "PAST ELECTROCARDIOGRAM" is checked, past electrocardiographic data can be attached as additional information. At this time, the past electrocardiographic data may be attachable by a simple selection operation from information stored in storage section 15 of request terminal 10.

The same applies to the other additional information, and it may be configured such that an automatic input from the information stored in storage section 15 of request terminal 10 is possible or an input can be performed by a simple selection operation. In addition, it may be configured such that the additional information of a group (for example, family, relatives, region, or the like) to which a living body for which a biological waveform is acquired belongs can be inputted or attached.

Further, FIG. 5 illustrates operation button B2 of "NEXT" for displaying interpretation request execution window W2 in FIG. 6 and operation button B3 of "CANCEL" for canceling an interpretation request.

When operation button B2 of "NEXT" is clicked in feedback item designation window W1, the processing proceeds to step S 14 described below. Further, when operation button B3 of "CANCEL" is clicked, the processing returns to step S12.

### (Step S14)

When operation button B2 of "NEXT" is clicked in feedback item designation window W1, request terminal 10 causes display screen 131 to display interpretation request execution window (interpretation request execution section) W2 (see FIG. 6) for executing an interpretation request.

FIG. 6 illustrates an example of display screen 131 of request terminal 10 in biological information interpretation support system 100, where display screen 131 displays interpretation request execution window W2. As illustrated in FIG. 6, interpretation request execution window W2 displays operation button B4 of "REQUEST INTERPRETATION" for executing an interpretation request, operation button B5 of "CANCEL" for canceling an interpretation request, and operation button B6 of "INTERPRETATION REQUEST DESTINATION" for selecting an interpretation request destination.

In interpretation request execution window W2, when operation button B4 of "REQUEST INTERPRETATION" is clicked, the processing proceeds to step S16 to be described later; when operation button B5 of "CANCEL" is clicked, the processing returns to step S12; and when operation button B6 of "INTERPRETATION REQUEST DESTINATION" is clicked, the processing proceeds to next step S15.

### (Step S15)

When operation button B6 of "INTERPRETATION REQUEST DESTINATION" indicated in FIG. 6 is clicked, request terminal 10 (request reception output section) causes display screen 131 to display request destination reception window (request destination reception section) W3 (see FIG. 7) for receiving designation of a request destination of an interpretation request.

FIG. 7 illustrates an example of display screen 131 of request terminal 10 in biological information interpretation support system 100, where display screen 131 displays request destination reception window W3. As illustrated in FIG. 6, request destination reception window W3 displays checkbox C1 of "a" and "PERFORM INTERPRETATION REQUEST TO EXTERNAL FACILITY" next to checkbox C1. When this checkbox C1 is checked, an interpretation request destination facility can be selected.

Below checkbox C1, interpretation request destination facility column F2 that displays a list of interpretation request destination facilities, selectable facility column F3 that displays a list of selectable facilities, operation button B7 of "ADD", and operation button B8 of "DELETE" are displayed.

Selectable facility column F3 displays a list of selectable facilities. By selecting one or a plurality of selectable facilities from the displayed list of selectable facilities and clicking operation button B7 of "ADD", the one or the plurality of selectable facilities is added to a list of interpretation request destination facilities, which is displayed in interpretation request destination facility column F2.

Interpretation request destination facility column F2 displays a list of interpretation request destination facilities. By selecting one or a plurality of interpretation request destination facilities from the displayed list of interpretation request destination facilities and clicking operation button B8 of "DELETE", the one or the plurality of interpretation request destination facilities is deleted from a list of interpretation request destination facilities, which is displayed in interpretation request destination facility column F2.

Here, the selectable facilities whose list is displayed in selectable facility column F3 may be facilities registered in advance or may include a facility(ies), if any, at which a patient has seen a doctor or the like previously.

Further, the selectable facilities whose list is displayed in selectable facility column F3 may be facilities that are in a state capable of receiving an interpretation request currently and are automatically selected from facilities registered in advance. For example, when request destination reception window W3 is displayed, statuses indicating whether reference terminals 41-1 to 41-n are in a state capable of receiving an interpretation request may be confirmed and selectable facility column F3 may display a list of selectable facilities.

With respect to the statuses described above, for example, statuses managed by applications in reference terminals 41-1 to 41-n may be confirmed or, instead of the statuses described above, it may be confirmed whether the applications are active. The same applies to a case where reference terminals 41-1 to 41-n are portable terminals such as smartphones or tablet terminals.

By confirming such statuses, it is possible to acquire, for example, in a case where a requester wishes an interpretation result to be returned promptly, the requester can acquire an interpretation request promptly by requesting a facility or an interpreter, which or who is in a state capable of receiving an interpretation request, to perform an interpretation, thereby reducing the time-related burden on the requester. In such a case, an interpretation request may be notified to an interpreter by using push notification or pop-up notification.

By performing the operation described above, one or a plurality of interpretation request destination facilities is selected, interpretation request execution window W2 indicated in FIG. 6 is displayed again upon completion of the selection, and the processing proceeds to step S16 to be described later by clicking operation button B4 of "REQUEST

### INTERPRETATION".

Here, although feedback item designation window W1, and interpretation request execution window W2 and request destination reception window W3 are displayed in this order, this order may be reversed. That is, the order of step S13, and steps S14 and S15 may be reversed and an order of step S14 → step S15 → step S13 may be employed. This order may be changed in accordance with reception of an interpretation request operation. Further, for example, in a case where the interpretation request destination facility is determined in advance, it is not necessary to select an interpretation request destination facility, and thus, step S15 may be skipped.

In the case of the order of step S14 → step S15 → step S13, for example, additional information input column F1 indicated in FIG. 5 may display additional information requested by the selected interpretation request destination facility.

### (Step S16)

When operation button B4 of "REQUEST INTERPRETATION" is clicked in interpretation request execution window W2 indicated in FIG. 6, request terminal 10 (transmission section) transmits an interpretation request to a selected interpretation request destination facility. The interpretation request is where designation of a feedback item is added to information on a biological waveform and an analysis result (information on a biological waveform or an analysis result), but the interpretation request also includes, if any, the additional information described above. At this time, request terminal 10 (transmission section) may transmit an interpretation request to reference terminals 41-1 to 41-n by email.

Note that, in a case where request terminal 10 is disposed at an MC office, dozens or hundreds of biological waveforms and/or analysis results may be inputted into request terminal 10. In such a case, it may be configured such that a plurality of biological waveforms and/or analysis results is collectively selected, then the input described above is performed, and interpretation requests are collectively transmitted to a selected interpretation request destination facility.

### (Step S17)

Reference terminals 41-1 to 41-n cause display screen 411 thereof to display an interpretation request window indicating an interpretation request transmitted from request terminal 10. Although illustration of the interpretation request window is omitted here, the interpretation request window displays, for example, a button(s) for returning acceptance/non-acceptance of an interpretation request (for example, an acceptance button and/or a non-acceptance button), or the like. At this time, reference terminals 41-1 to 41-n may cause display screen 411 thereof to perform a pop-up display of the interpretation request window to notify an interpreter that an interpretation request has come. The same applies to a case where reference terminals 41-1 to 41-n are portable terminals such as smartphones or tablet terminals.

### (Step S18)

When the button(s) for returning acceptance/non-acceptance of an interpretation request (for example, an acceptance button and/or a non-acceptance button), or the like is/are clicked by an interpreter at an interpretation request destination facility, reference terminals 41-1 to 41-n return acceptance/non-acceptance of the interpretation request to request terminal 10.

### (Step S19)

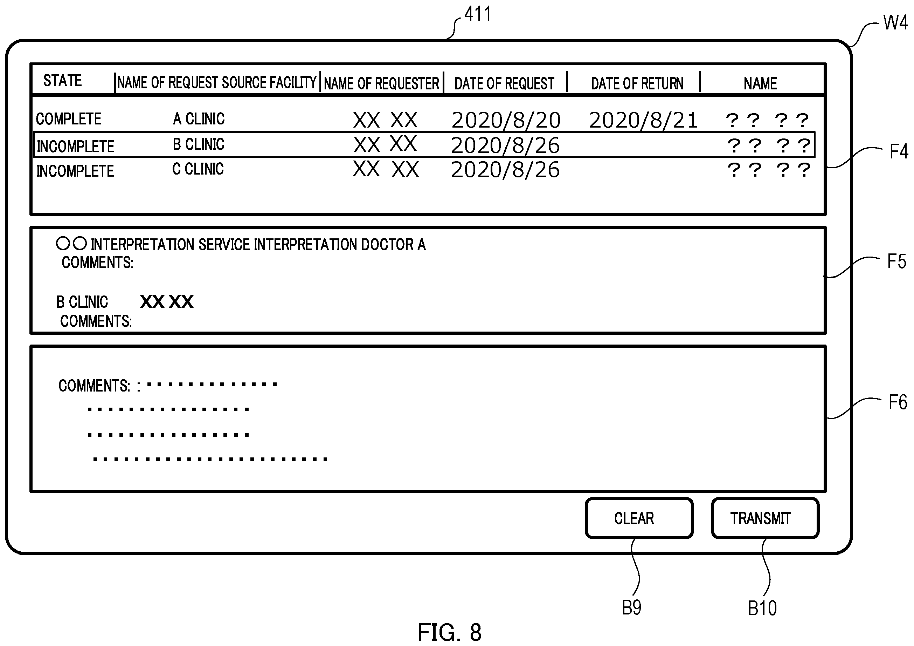

Upon accepting an interpretation request, reference terminals 41-1 to 41-n cause display screen 411 thereof to display interpretation result input window (interpretation result input section) W4 (see FIG. 8).

FIG. 8 illustrates an example of display screen 411 of reference terminals 41-1 to 41-n in biological information interpretation support system 100, where display screen 411 displays interpretation result input window W4.

As illustrated in FIG. 8, interpretation result input window W4 displays request source facility column F4 that displays a list of request source facilities with relevant information, interpretation display column F5 that displays contents of an interpretation result from an interpretation request destination facility and contents of an interpretation request from a request source facility, and input column F6 for an interpretation result.

FIG. 8 illustrates display screen 411 in a reference terminal of ∘∘ interpretation service in a state in which an interpretation result for an interpretation request from B clinic has been inputted into input column F6. Accordingly, the row of B clinic is selected in request source facility column F4, and interpretation display column F5 displays comments (interpretation result) of interpretation doctor A of ∘∘ interpretation service and comments (interpretation request) of requester ××× of B clinic.

Further, interpretation result input window W4 displays operation button B9 of "TRANSMIT" for transmitting an interpretation result inputted into input column F6, and operation button B10 of "CLEAR" for erasing an interpretation result inputted into input column F6.

Here, interpretation doctor A of ∘∘ interpretation service performs an interpretation and consultation for a biological waveform, or the like, based on the interpretation request of requester ××× of B clinic, performs an input thereof into input column F6, and clicks operation button B9 of "TRANSMIT". When the interpretation request from the requester includes an item of consultation for an electrocardiographic waveform, interpretation doctor A inputs, for example, recommendation for using a Holter electrocardiograph, recommendation for performing an echocardiographic examination, recommendation for seeing a doctor at a specialized medical institution, or the like, into an interpretation result. Note that, a code may be used for the interpretation result when there is a coded name of disease, comments, or the like.

In a case where an interpretation request includes additional information, an interpreter interprets a biological waveform by taking the additional information into consideration as well. For example, in a case where additional information includes a past electrocardiogram, an interpreter performs an interpretation by comparing an electrocardiogram with the past electrocardiogram. For example, it is known that QT on an electrocardiogram is prolonged as a risk involving taking a prescribed drug, but it is possible to evaluate whether the QT prolongation is caused by taking a drug or not, by comparing an electrocardiogram with the past electrocardiogram.

Further, for example, in a case where additional information includes an electrocardiogram of a relative of a patient, an interpreter performs an interpretation by comparing an electrocardiogram of the patient with the electrocardiogram of the relative. For arrhythmia, myocardial infarction, or the like, it is recommended that hearing of the medical history of a relative(s) of a patient be performed for risk stratification thereof. However, a patient himself/herself does not necessarily grasp the medical history of a relative(s) of the patient. Further, it is not necessarily possible to confirm the medical history of a relative(s) of a patient at the time of hearing. When such information is accumulated in, for example, storage section 15 and can be included in an interpretation request as additional information, it is possible to perform an interpretation by taking the medical history of a relative(s) of a patient into consideration and to enhance the quality of medical practice.

Further, for example, in a case where additional information includes a past blood pressure and pulse waveform, an interpreter performs an interpretation by comparing a blood pressure and pulse waveform with the past blood pressure and pulse waveform. For example, in the tendency of lower extremity constriction, a post-operative evaluation, or the like, absolute values of blood pressure values or the like may not allow the tendency thereof to be grasped. In such a case, it is possible to perform a disease state evaluation or a post-operative evaluation by comparing a blood pressure and pulse waveform with a past blood pressure and pulse waveform and evaluating changes in waveforms.

Further, reference terminals 41-1 to 41-n may have a drawing function capable of causing display screen 411 to display a biological waveform and an analysis result as illustrated in FIGS. 3 and 4 and performing drawing indicating a portion that serves as a basis for the interpretation result described above.

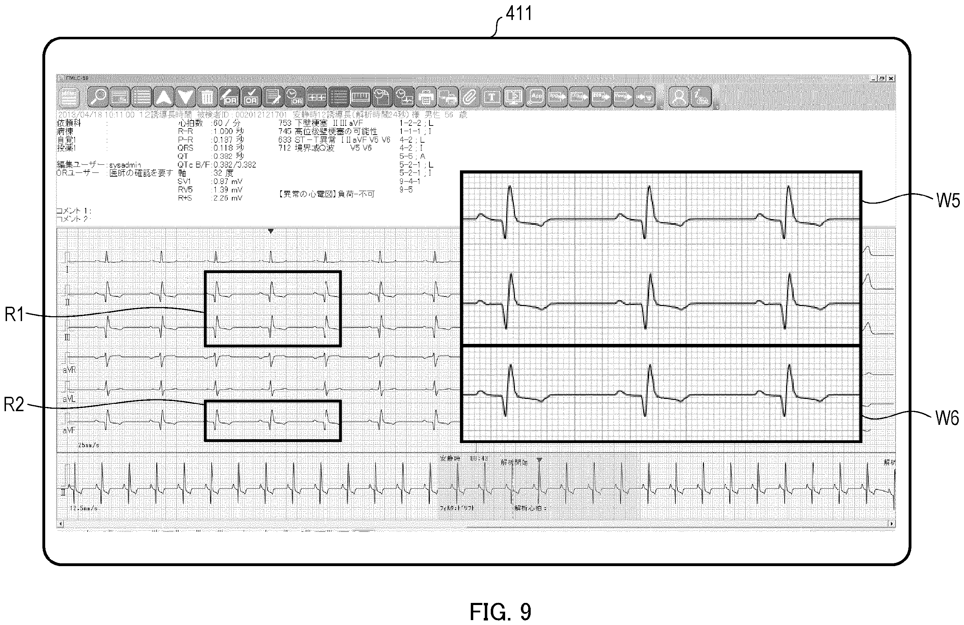

Further, reference terminals 41-1 to 41-n may have an enlargement and reduction function capable of enlarging or reducing a biological waveform in order to view the biological waveform in detail. For example, as illustrated in FIG. 9, in a case where an interpreter wishes to enlarge specific portions in an electrocardiographic waveform, the interpreter selects the portions with selection regions R1 and R2, then pop-up displays of enlarged windows W5 and W6 indicating enlarged views of the portions surrounded by selection regions R1 and R2 are performed. Then, in a case where the interpreter wishes to return an interpretation result of "suspected atrial fibrillation" to the requester, the interpreter selects, as a basis for the interpretation result, a specific induction in the electrocardiographic waveform and specifies the presence or absence of a P wave or the like by the drawing function or the enlargement and reduction function.

Further, in a case where there is a plurality of interpretation results (for example, names of diseases or the like), the drawing function changes marks and colors thereof so as to correspond to the respective interpretation results, whereby the portions corresponding to the respective interpretation results may be distinguishably displayed with marks and colors.

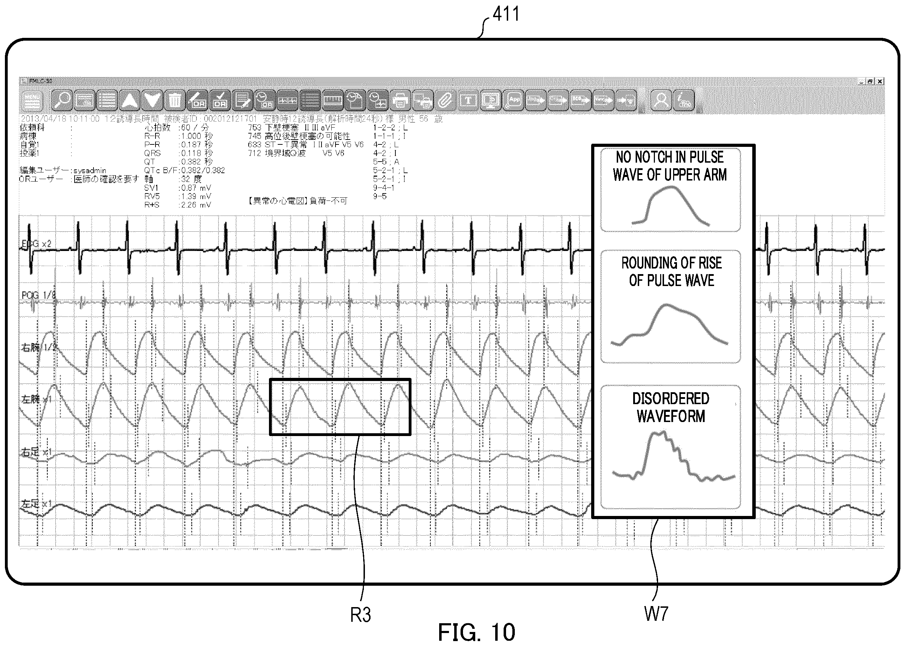

Further, in a case where a biological waveform to be interpreted includes a heart sound waveform, a blood pressure and pulse waveform, or the like, a pop-up display of window W7 that indicates a comparative example(s) for compari son with an arbitrary portion in a heart sound waveform or a blood pressure and pulse waveform, where the portion is selected with selection region R3 and is surrounded by selection region R3, may be performed as illustrated in FIG. 10.

Further, in a case where a plurality of interpreters performs an interpretation at one interpretation destination facility, for example, the interpretation may be performed step by step by a medical laboratory scientist through a medical specialist, and a doctor with the ultimate responsibility may approve an interpretation result.

### (Step S20)

When operation button B9 of "TRANSMIT" is clicked, reference terminals 41-1 to 41-n transmit an interpretation result to request terminal 10. At this time, reference terminals 41-1 to 41-n may transmit the interpretation result to request terminal 10 by email.

### (Step S21)

Request terminal 10 causes display screen 131 to display a transmitted interpretation result. At this time, the transmitted interpretation result is displayed so as to be tied to the corresponding biological waveform and/or analysis result. Further, the transmitted interpretation result is stored in storage section 15 so as to be tied to the corresponding biological waveform and/or analysis result. Note that, at this time, request terminal 10 may cause display screen 131 thereof to perform a pop-up display of the interpretation result to notify the requester that the interpretation result has been returned. The requester can determine a more accurate diagnosis of a patient and a treatment policy going forward by confirming the displayed interpretation result tied to the biological waveform and/or analysis result.

Note that, request terminal 10 can cause an interpretation result to be printed by a printer whose illustration is omitted. Further, request terminal 10 may have the function of editing an interpretation result such that the interpretation result can be used for an application required for seeing a doctor at a specialized medical institution, or the like.

Further, request terminal 10 may have the function of returning acceptance for an interpretation result in a case where the requester is convinced of the interpretation result, and of transmitting an interpretation request to any other interpretation request destination or interpreter in a case where the requester is not convinced of the interpretation result.

As described above, request terminal 10 is configured such that a requester can easily select contents of an interpretation request when requesting an interpretation. Further, request terminal 10 is configured such that a requester can easily select a request destination when requesting an interpretation. In this way, request terminal 10 can support a request for an interpretation of a biological waveform. As a result, a requester no longer hesitates to request an interpretation and the psychological burden on the requester can be reduced.

Further, an MC office can easily request an interpreter outside the MC office to perform an interpretation by using biological information interpretation support system 100 even without securing any interpreter within the MC office, thereby reducing costs for securing an interpreter. Further, even when a plurality of interpreters is secured within an MC office, it may be difficult to maintain the quality of interpretation because of differences in interpretation abilities due to experiences of the interpreters, or the like. In such a case, it is possible to achieve homogenization of interpretations and quality maintenance by using, for example, biological information interpretation support system 100 to select an interpreter having at least predetermined years of experience and to request the interpreter to perform an interpretation.

### <Variation 1>

In the embodiment described above, request terminal 10 in biological information interpretation support system 100 functions as the biological information interpretation support apparatus of the present invention. However, it may also be configured such that biological information interpretation support system 100 is provided with a server and the server has some functions of request terminal 10.

For example, it is configured such that a server such as a cloud server is connected to network 30 and the server performs a part related to the communication between request terminal 10 and reference terminals 41-1 to 41-n.

Further, it may be configured such that a server has the automatic analysis function for biological information (biological waveform) instead of the measurement apparatuses (such as electrocardiograph 21) and request terminal 10.

Further, in the embodiment described above, a configuration in which the ratio of request terminal 10 to reference terminals 41-1 to 41-n is one to n has been indicated as an example, but the configuration can be easily changed, by providing the server described above, to a configuration in which the ratio of m request terminals 10 (where m is a natural number) to reference terminals 41-1 to 41-n is m to n.

Further, it may be configured such that required information can be referred to from reference terminals 41-1 to 41-n by providing a database server connected to network 30 and accumulating the information described above, which is stored in storage section 15, in the database server.

In the embodiment described above, the biological waveform has been exemplified as the biological information, but the biological information is not limited to the biological waveform, but may be, for example, information on a numerical value forming a biological waveform (for example, a numerical value of a specific portion in a biological waveform) or may be information illustrating a dynamic state of a living body or a time-series change in a living body.

In addition, any of the embodiment described above is only illustration of an exemplary embodiment for implementing the present invention, and the technical scope of the present invention shall not be construed limitedly thereby. That is, the present invention can be implemented in various forms without departing from the gist or the main features thereof.

The disclosure of Japanese Patent Application No. 2020-191771, filed on November 18, 2020, including the specification, drawings and abstract, is incorporated herein by reference in its entirety.

### Reference Signs List

10 Request terminal
11 Control section
12 Acquisition section
13 Operation display section
14 Communication section
15 Storage section
21 Electrocardiograph
22 Blood pressure and pulse wave meter
23 Phonocardiograph
30 Network
41-1 to 41-n Reference terminal
100 Biological information interpretation support system

## Claims

1. A biological information interpretation support apparatus, comprising:
an information output section that causes a display screen to display biological information and/or information on an analysis result of the biological information;
a request reception output section that causes the display screen to display an interpretation request reception section, the interpretation request reception section receiving an interpretation request operation for the biological information;
a designation reception section that receives designation of a feedback item, the feedback item being required as an interpretation result of the biological information during the interpretation request operation; and
a transmission section that transmits an interpretation request in which the designation of the feedback item is added to the biological information and/or the information on the analysis result.

2. The biological information interpretation support apparatus according to claim 1, wherein the designation reception section causes a feedback item designation section to be displayed, the feedback item designation section receiving the designation of the feedback item.

3. The biological information interpretation support apparatus according to claim 2, wherein the designation reception section causes the feedback item designation section to display the feedback item that is designatable.

4. The biological information interpretation support apparatus according to claim 2, wherein the designation reception section causes the feedback item designation section to be displayed after reception of the interpretation request operation.

5. The biological information interpretation support apparatus according to claim 4, wherein the designation reception section causes the feedback item designation section and a request destination reception section to be sequentially displayed in accordance with the reception of the interpretation request operation, the request destination reception section receiving designation of a request destination of the interpretation request.

6. The biological information interpretation support apparatus according to claim 5, wherein the designation reception section causes the request destination reception section to display a plurality of the request destinations and receives the designation of the request destination among the plurality of request destinations having been displayed, the plurality of request destinations being in a state in which the plurality of request destinations is allowed to receive the interpretation request.

7. The biological information interpretation support apparatus according to claim 2, wherein the designation reception section causes the feedback item designation section to display an input column that receives an input of additional information including at least one of a history, a symptom, a prescription, and/or a chief complaint and/or the additional information of a living body in a group to which another living body belongs, the history, the symptom, the prescription, and the chief complaint being related to the other living body for which the biological information is acquired.

8. A biological information interpretation support method, comprising:
causing a display screen to display biological information and/or information on an analysis result of the biological information;
causing the display screen to display an interpretation request reception section, the interpretation request reception section receiving an interpretation request operation for the biological information;
receiving designation of a feedback item, the feedback item being required as an interpretation result of the biological information during the interpretation request operation; and
transmitting an interpretation request in which the designation of the feedback item is added to the biological information and/or the information on the analysis result.

9. A biological information interpretation support program, which causes a computer to execute the biological information interpretation support method according to claim 8.
